# EUROPEAN PATENT APPLICATION

(11) **EP 1 188 832 A1**
(43) Date of publication of application: **20.03.2002**
(21) Application number: 00203115.1
(22) Date of filing: 08.09.2000
(51) Int. Cl.: C12N 15/62, G01N 33/574, C07K 14/16, C07K 14/01, C12N 15/34, C12N 1/21, C12N 15/87, A61K 38/16, A61P 35/00, A61P 37/00

(54) **A delivery method for the tumor specific apoptosis inducing activity of apoptin**

(71) Applicant: Leadd B.V., 2333 AL Leiden (NL)
(72) Inventor: Noteborn, Mathieu Hubertus Maria, 2352 EH Leiderdorp (NL); Zhang, Ying-Hui, 2334 ET Leiden (NL); Voorhoeve, Pieter Mathijs, 1091 TS Amsterdam (NL); Leliveld, Sirik Rutger, 2311 TS Leiden (NL)
(74) Representative: Prins, Adrianus Willem

(57) **Abstract**

The invention relates to the field of apoptosis. The invention provides novel therapeutic substances, for example novel therapeutic proteinaceous compounds that can contain apoptin alone or jointly with other proteinaceous protein or protein fragments, especially in those cases when cells are derailed such as cancer-, auto-immune-derived cells.

## Description

### Brief description of the invention

The invention relates to a method for inducing apoptosis specifically in transformed or malignant cells by introduction of protein with apoptin (vp3) like activity into these cells.

### Background of the invention

Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996).
The apoptotic process can be initiated by a variety of regulatory stimuli (Wyllie, 1995, White 1996, Levine, 1997). Changes in the cell survival rate play an important role in human pathogenesis, e.g. in cancer development and auto-immune diseases, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Certain transforming genes of tumorigenic DNA viruses can inactivate p53 by directly binding to it. Examples of such agents are the E6 protein from oncogenic subtypes of the Human Papiloma Virus and the large T antigen of the tumor DNA virus SV40 it (Teodoro, 1997).

Another example of the emergence of a strong resistance to various apoptosis-inducing chemotherapeutic agents in (leukemic) tumors is the association of a high expression level of the proto-oncogene Bcl-2 or Bcr-abl with reduced sensitivity of these tumors to therapy (Hockenberry 1994, Sachs and Lotem, 1997).

Apoptin (also called vp3; the terms may be used interchangeable herein) is a small protein derived from chicken anemia virus (CAV; Noteborn and De Boer, 1995, Noteborn et al., 1991, Noteborn et al., 1994; 1998a). By means of transient transfection using plasmids encoding apoptin it was shown that apoptin can induce apoptosis in human malignant and transformed cell lines, but not in non-transformed human cell cultures. In vitro, also by means of transient transfections, apoptin fails to induce programmed cell death in normal lymphoid, dermal, epidermal, endothelial and smooth-muscle cells. However, when normal cells are transformed they become susceptible to apoptosis induced by apoptin. In normal cells, apoptin was found predominantly in the cytoplasm, whereas in transformed or malignant cells i.e. characterized by hyperplasia, metaplasia or dysplasia, it was located in the nucleus (Danen-van Oorschot, 1997 and Noteborn, 1996).

Long-term expression of apoptin in normal human fibroblasts revealed that apoptin has no toxic or transforming activity in these cells (Danen-van Oorschot, 1997 and Noteborn, 1996). The fact that apoptin does not induce apoptosis in normal human cells, at least not in vitro, suggests that a toxic effect of apoptin treatment in vivo will be very low. Noteborn and Pietersen (1998) and Pietersen et al. (1999) have provided evidence that adenovirus expressed apoptin does not have an acute toxic effect in vivo, whereas in nude mice it has a strong anti-tumor activity. Further evidence of a lack of toxicity in vivo comes from transgenic mice which express apoptin from an MHC-I promoter and which have no observable abnormalities (Noteborn and Zhang, 1998).

Of importance in the treatment of tumors that have become resistant to chemo or radiation therapy is that apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn, 1996). In addition, it appears, that even premalignant, minimally transformed cells, may be sensitive to the death-inducing effect of apoptin (Noteborn and Zhang, 1998).

Thus, to further enlarge the array of therapeutic anti-cancer or anti-auto-immune-disease compounds available in the art, additional therapeutic proteins are desired. The invention provides novel therapeutic substances, for example novel therapeutic proteinaceous compounds that can contain apoptin alone or jointly with other proteinaceous substances or protein fragments, especially in those cases when cells are derailed such as cancer-derived or auto-immune-derived cells.

In a first embodiment, the invention provides a proteinaceous apoptin-like containing substance that can induce apoptosis in a tumor-specific way. Apoptin protein exerts its tumor-specific cytotoxicity when administered to cells.

In particular, the detailed description provides evidence that micro-injection of either an apoptin fusion protein, such as non-denatured Maltose-binding-protein (MBP)-apoptin fusion proteins or a His-tagged apoptin protein, both produced in E. coli cells and purified using affinity-purification, result in apoptosis induction of human tumorigenic/transformed cells but not of normal human primary cells. Furthermore, the his-tagged protein was purified under denaturing conditions and subsequently allowed to refold. The proteins showed specific activity, showing that both non-denatured protein and refolded protein can be used.

The invention also describes that the activity and behavior of the recombinant proteins is similar to the activity of apoptin protein produced by transcription and translation of the apoptin DNA without protein fusion or tag. This indicates that the apoptin DNA is not necessary for apoptin like activity other than as a template for transcription by the cell. The invention shows furthermore that the presence of a fusion protein on the N-terminus of apoptin, or of an six his-tag on the C-terminus, does not disturb the specificity or the activity of apoptin. Also the specific localization to the nucleus in transformed cells is observed with recombinant apoptin protein, indicating that the localization of apoptin protein can be used as a diagnostic marker for a transformed/tumorigenic phenotype of tissue samples or cells cultured in vitro.

The invention also describes due to its ability to differentiate between normal and transformed cells that recombinant apoptin protein harbors potential activity for the destruction of tumor cells, or other hyperplasia, metaplasia or dysplasia, with minimal or no toxicity to normal tissue. Moreover, apoptin protein or derivatives of it such as apoptin-fusion proteins will also be effective against tumors which have become resistant to (chemo)-therapeutic induction of apoptosis, due to the lack of functional p53 and (over)-expression of Bcl-2 and other apoptosis-inhibiting agents.

This invention also describes another example of an effective anti-tumor therapy based on apoptin-derived proteinaceous substances. To eradicate a tumor, it is imperative that all cells of the tumor, including its potential mini-metastases, are reached by an anti-tumor agent. Until now this has been a bottleneck in the development of an efficient anti-tumor therapy based on gene delivery.

The invention describes a method allowing direct introduction of apoptin protein into cells achieved in vitro and in vivo by coupling this effector protein (henceforth referred to as the cargo) to a protein transduction domain. The first description of the capability of certain proteins to cross cell membranes was given independently by Green and Loewenstein (1988) and Frankel and Pabo (1988), for the HIV TAT protein. Henceforth it was shown that synthetic peptides containing the amino acids 48 to 60 derived from the HIV TAT protein could transduce into cells (Vives et al., 1997). This ability can be conferred in trans by chemical cross-linking the TAT-derived protein transduction domain to the cargo protein, enabling proteins as large as 120 kDa to be delivered intra-cellularly, in vitro as well as in vivo (Fawell et al., 1994). Other transduction domains have been described in the Antennapedia protein from Drosophila melanogaster (Derossi et al., 1998) as well as in other homeodomain proteins, the Herpes Simplex Virus vp22 protein (Elliott et al., 1999), and several synthetic peptides (Lindgren et al., 2000). The HIV-TAT-mediated process does not depend on endocytosis and is not mediated through a cellular receptor. This explains the remarkable universality that is seen; the proteins can be transduced into all cells tested thus far (Schwarze and Dowdy, 2000). An efficient method based on the HIV TAT peptide has been developed to make recombinant proteins that can be transduced both in vitro and in vivo. Significantly, when administered in vivo, all tissues, including the brain, can be targeted with a recombinant protein (Schwarze, 1999) using this system.

Another class of delivery method is based on the hydrophobic core region of signal peptides (Hawiger, 1999). The cellular import of fusions or conjugations between these transduction domains and a cargo are concentration and temperature sensitive. It is, however, cell type independent and a whole range of cells have been shown to be susceptible to internalization of cargo mediated by this class of transduction domains.

In general for the transduction protein technology the drawback is that it has not yet been possible to target a specific (tumor) cell compartment with this system. The lack of tumor specific targeting has thus far prevented the development of an efficient anti-tumor therapy, which uses protein transduction.

In a preferred embodiment, the present invention describes a method, which circumvents this drawback. Transduced cells, which take up apoptin-derived protein are only undergoing cell death when they are of a transformed or malignant nature and stay alive when they are normal. This means that the use of apoptin as part of a transductioncapable proteinaceous substance will make it a potential anti-tumor agent.

The invention describes another way to introduce (recombinant) apoptin protein into cells, which is by fusion of the protein with a ligand. Receptor mediated internalization then results in the uptake of the fusion protein. Examples for such apoptin fusion proteins are based on the Epidermal Growth Factor (EGF), and apoptin-fusion proteins containing ligands binding to the hormone receptors such as thyroid receptors.

All these methods hinge on the activity of the recombinant or purified cargo-protein in the target cell. In particular, the invention shows that apoptin protein produced in various ways retains its specific tumor killing ability, and thus opens the way to combine the generalized delivery of protein transduction with the specific anti-tumor activity of apoptin, which results in a new method by which transformed or malignant cells can be eradicated.

Thus, the invention describes several examples of apoptin-derived proteinaceous substances such as MBP apoptin cross-linked to a transduction domain such as TAT can be applied, and also his-tagged apoptin protein containing a transduction domain as a fusion can be applied, either as a non-denatured protein or in denatured renatured format.

Furthermore, the invention describes other transduction domains as mentioned above can be envisaged, where all share the capacity to introduce the apoptin protein into tumor cells and normal cells alike. Fusion to a ligand may specifically target apoptin to one cell type, but the tumor specificity of apoptin will be pivotal for therapeutic applications with minimal collateral damage to normal cells. As an example, EGF-targetted apoptin could be introduced into all EGF-receptor expressing cells. Apoptin will, however, destroy only the tumor cells.

The invention provides the application of cell-permeable protein as a drug being much more safe in the long term than gene-therapy approaches possibly causing genetic alterations resulting in diseases such as cancer.

Use as provided by the invention is particularly useful from a therapeutic viewpoint. The invention provides herewith a pharmaceutical composition comprising a proteinaceous substance capable of providing tumor specific apoptosis. The said proteinaceous substance comprises apoptin or functional equivalent or functional fragment thereof. More preferably, the proteinaceous substance further comprises a means to deliver said apoptin to a cell, a fusion protein and/or a tag. The invention further provides evidence that the proteinaceous substance according to the invention can be either non-denatured or refolded.

Such a pharmaceutical composition is in particular provided for the induction of apoptosis, for example wherein said apoptosis is p53-independent, for the treatment of a disease where enhanced cell proliferation or decreases cell death is observed comprising treating said individual with a pharmaceutical composition according to the invention. Therefor, these compositions are essential for new treatments, but also for diagnosis of diseases with aberrancies in the apoptotic process, such as cancer and auto-immune diseases.

In the field of diagnosis the invention provides a method for detecting the presence of cancer cells or cells that are cancer prone in a sample of cells comprising providing cells in said sample with a proteinaceous substance capable of providing tumor specific apoptosis according to the invention, culturing said cells and determining for example, the percentage of apoptosis of cells in said sample. The said proteinaceous substance comprises apoptin or functional equivalent or functional fragment thereof. More preferably, the proteinaceous substance further comprises a means to deliver said apoptin to a cell, a fusion protein and/or a tag. The invention further provides evidence that the proteinaceous substance according to the invention can be either non-denatured or refolded.

The invention will be explained in more detail in the following description, which is not limiting the invention.

### Detailed description of the invention

The invention provides a system to produce and deliver intracellularly recombinant proteinaceous substances comprising apoptin or functional equivalent or functional fragments there of, or recombinant proteinaceous substances with apoptin-like activity.

The invention further provides for the addition of further optional modular peptides. This can include an epitope tag, allowing easy detection and immunoprecipitation without direct steric hindrance of associations of apoptin with cellular proteins.

The invention provides or describes all steps needed for the production of recombinant apoptosis inducing agent apoptin, or derivatives of apoptin that have a similar tumor specificity. The recombinant protein can be produced in E. coli, insect cells by means of a baculovirus-based vector, or in yeast strains (such as Pichia pastoris).

The invention provides evidence that the apoptin or apoptin-like proteinaceous substance does not need to be folded properly in the producer cell, enabling the production of recombinant apoptin or apoptin-like proteinaceous substances in transgenic plant cells, for mass production.

The invention proposes a modified metal affinity tag for optimal purification of the recombinant protein. By using a double His-tag next to the transduction domain, a significantly better binding to Nickel beads is achieved, resulting in the possibility to wash the recombinant apoptin or apoptin-like proteinaceous substances under very stringent conditions resulting in an optimal purification of apoptin or apoptin-like proteinaceous substances.

The invention describes the use of a transduction domain fused to recombinant apoptin protein. This domain allows the recombinant protein to pass through the cellular membrane. This domain can consist of a transduction domain derived from HIV TAT, or of any other known transduction domain.

The invention describes the use of CAV-derived vp3 (apoptin) proteinaceous substance as part of a fusion protein, or derivatives of apoptin proteinaceous substances that are selected on their ability to specifically induce apoptosis in tumor cells. The invention also provides a therapy for cancer, autoimmune diseases or related diseases which is based on apoptin or apoptin-like proteinaceous substances.

The invention also provides a method to remove aberrant cells in their first stages of transformation and premalignant lesions.

The invention describes a research tool based on apoptin or apoptin-like proteinaceous substances to enravel apoptosis and transformation pathways or as diagnostics for determining the tumorigenic status of patient material.

### Experimental part

### 1. Production and purification of MBP-apoptin (MBP-vp3) protein

### 1.1 MBP-vp3 expression construct

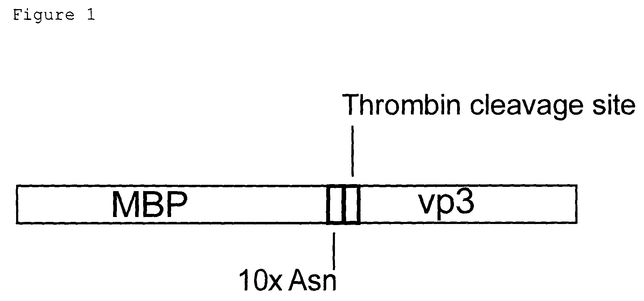

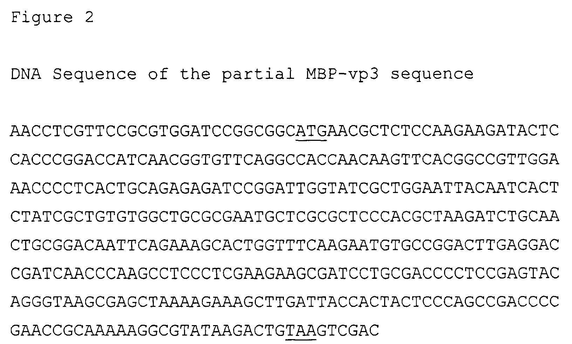

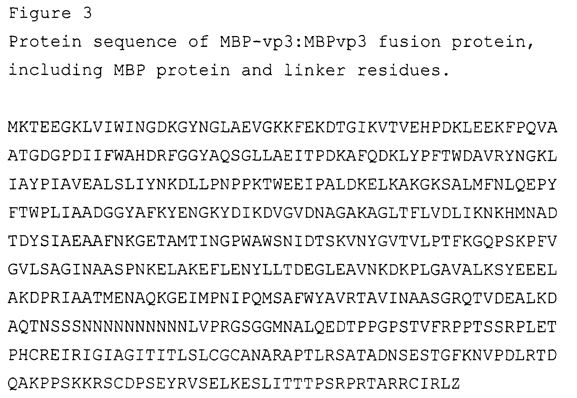

The vp3 gene was fused in frame in a bacterial expression vector encoding the Maltose-binding protein (MBP), a 10 Asn linker and a Thrombin-cleavage site. The expression system is based on a modified pMal-c2 plasmid vector (New England Biolabs, USA), in which the factor Xa site has been replaced by a thrombin-cleavage site. This modified vector was named pMalTB. A PCR fragment consisting of the complete apoptin or vp3 sequences and at the 5'-end a BamHl and at the 3'-end a SalI site, was cloned in pMalTB. The resulting fusion product consists of a N-terminal MBP moiety that is separated from the vp3 part by a 10-Asn linker and a thrombin-cleavage site as shown in Figure 1. The DNA and protein sequence of the fusion product is given in Figure 2 and Figure 3, respectively. The resulting plasmid is called pMBP-vp3 and the proteinaceous substance encoded by this plasmid is designated MBP-vp3. The correct sequence of the essential parts of the MBP-vp3 construct was confirmed by means of the Sanger method (Sanger et al., 1973) and carried out by Base-Clear, Leiden, The Netherlands.

### 1.2 Expression and purification of MBP-vp3

The plasmid pMBP-vp3 was transformed in bacteria derived from strain BL21(DE3), and initial expression studies showed that MBP-vp3 protein constitutes roughly 10% of the soluble cytoplasmatic protein, after 3 hours induction with 1mM IPTG. Purification was carried out on amylose beads at pH 7.4 and 1M NaCl. Subsequently, elution in buffer containing 20mM HEPES 8.0, 50mM NaCl, 1mM EDTA, 1mM DTT, 10mM maltose, yielded about 100mg protein per liter of bacterial culture. This purified protein was loaded on a UNO-31 chromatography column (Biorad), and the fractions that elute at 400-500mM NaCl, 20mM HEPES pH7.4, 1mM EDTA were pooled, dialyzed against PBS and concentrated with Millipore UltraFree spin filters.

The negative control preparation being the Maltose binding protein (MBP) was produced and purified in the way as been described for MBP-vp3.

### 2. Expression and purification of His-tagged apoptin

### 2.1 His-tagged vp3 construct

Vp3 lacking a stop codon was cloned in the NdeI site and NotI site of the IPTG-inducible bacterial expression plasmid pET22b, which provides in frame a 6-histidine tag and a stop codon. The essential regions of the final pVp3H6 DNA construct were sequenced according to the Sanger method and carried out by Base Clear, Leiden, The Netherlands. The map of the essential regions of pVp3H6 is shown in Figure 4a and the DNA and protein sequence of the his-tagged vp3 (apoptin) region is given in Figure 5 and 6, respectively.

### 2.2. Vp3H6 expression and purification

The Vp3H6 construct was transformed in BL21(DE3) bacteria (Novagen) and a colony was grown at 37°C to an OD600 of ca. 0.6. Expression was then induced by adding 1 mM IPTG and the cells were grown for an additional 3 hrs. After harvesting by centrifugation, the cells were lysed in a Bead-Beater (Biospec Inc.) in lysis buffer (containing 50 mM NaHEPES pH 7.4, 100 mM NaCl, 1 mM EDTA, 1 mM DTT and protein inhibitors (Complete, Boehringer)). The inclusion bodies were harvested by centrifugation and made soluble by suspending in Solubilisation Buffer (containing 50 mM HEPES pH 7.4, 20 mM Glycine, 1 mM EDTA, 10 mM DTT, 8 M Urea). The cleared supernatant was loaded directly on UNO-S12 (Biorad), pre-equilibrated with: 20 mM KPO₄, 5 mM Imidazole, 6 M urea, 1 mM GSH. The Vp3H6 protein was eluted with a NaCl gradient (0-1 M NaCl at 3 ml/min with a total volume of 200 ml). Vp3H6 eluted between 400 and 650 mM NaCl. It was loaded directly on Ni-NTA (Qiagen) (pre-equilibrated in 20 mM KPO₄ pH 7.4, 5 mM Imidazole, 500 mM NaCl, 6 M urea at 4C). Next, the column was washed with 20 mM KPO₄ pH 7.4, 20 mM Imidazole, 500 mM NaCl, 6 M GuHCl. The GuHCl was removed by washing with 20 mM KPO₄ pH 7.0, 400 mM NaCl, 2 mM MgCl2, 1 mM GSH, and Vp3H6 protein eluted with 20 mM KPO₄ pH 7.4, 400 mM NaCl, 500 mM Imidazole, 2 mM MgCl2. The Vp3H6 protein containing peak fractions were pooled and 5 mM EDTA was added to remove Nickel traces. The sample was dialysed (1 volume to 200) to 20 mM KPO₄ pH 6.5, 400 mM NaCl, 2 mM MgC12, 1 mM DTT. Finally, the Vp3H6 protein was concentrated on Centricon YM3 filters (Millipore) to at least 7 mg/ml.

### 3. Biological activity of MBP-yp3 protein and his-tagged apoptin protein.

### 3.1 Tumor-specific induction of apoptosis by proteinaceous apoptin (vp3) substances

To assay the ability of apoptin protein to specifically induce apoptosis in tumor cells a micro-injection system was set up. Human osteosarcoma-derived Saos-2 tumor cells and normal human diploid VH10 primary cells were cultured on glass cover slips. The cells were micro-injected in the cytoplasm with protein MBP-vp3, Vp3H6, or MBP alone at 3mg/ml using an Eppendorf micro-injector with the injection-pressure condition of 0.5 psi. The cells were co-injected with Dextran-Rhodamine (MW: 70 kDa; Molecular Probes, Leiden, The Netherlands) to be able to later identify injected cells. The cells were incubated at 37°C after injection until the cells were fixed with formaldehyde-methanol-acetone. Presence of MBP-apoptin or his-tagged apoptin protein was determined with immuno-histochemistry with antibodies directed against MBP (monoclonal mouse anti-MBP-clone R29; Zymed Laboratories, Inc. and polyclonal rabbit anti-MBP- clone C18/sc-808; Santa Cruz Biotechnology, Inc.) and/or against apoptin (vp3; anti-VP3C). Apoptosis was counted as abnormal nuclear morphology after counter-staining with DAPI and examination with an immunofluorescence microscope (Telford et al., 1992).

The results show that both MBP-vp3 and Vp3H6 protein were able to induce rapid apoptosis in human tumor cells (within 3-6 hours , but did not induce apoptosis in normal human cells. The MBP control protein did not induce apoptosis in any of the cell lines under these conditions. The fact that both proteinaceous apoptin (vp3) substances can induce apoptosis in human tumor cells lacking p53 implies that both proteinaceous apoptin substances induce apoptosis where known anti-cancer therapies fail.

Furthermore, the apoptin-characteristic tumor-specific cellular localization was observed for both MBP-vp3 as well as for Vp3H6 protein. In the human tumorigenic Saos-2 cells, both MBP-vp3 and Vp3H6 apoptin proteinaceous substance was predominantly located within the nucleus of pre-apoptotic cells. Somewhat later, these MBP-vp3- and Vp3H6-positive cells underwent apoptosis. In normal non-transformed human VH10 cells, both MBP-vp3 and Vp3H6 are mainly localized in cytoplasmic structures as has been described for apoptin by Danen-Van Oorschot et al. (1997).

In conclusion, exogenous produced proteinaceous apoptin substances harbor a tumor-specific apoptosis activity, which can be the base of a novel anti-cancer therapy.

### 3.2 Apoptosis induction in the absence of de novo protein production.

The following experiment shows an example of an application of the proteinaceous apoptin substance for studying the tumor-specific apoptosis pathway, which can be induced by apoptin. Saos-2 cells were incubated with transcription inhibitors cycloheximide (20 ug/ml or actinomycin D (10 ug/ml) or translation inhibitors puromycin (10 ug/ml) and emetin (10 ug/ml), or without these inhibitors. The transcription- as well as the translation-inhibitors were obtained from the company Sigma, USA. The inhibition efficacy of the various transcription- and/or translation-inhibitors was proven to be accurate in Saos-2 cells by micro-injecting Saos-2 cells with a plasmid expressing the Green-fluorescence protein (GFP). In all cases no GFP protein could be produced by the Saos-2 cells when they were treated with any of the 4 translation- or transcription-inhibitors. In contrast, the Saos-2 cells micro-injected with the GFP-encoding plasmid produced the GFP in clearly detectable amounts as visualized by direct fluorescence techniques.

For each type of inhibitor, 2 dishes with Saos-2 cells were used. As positive control, Saos-2 cells without inhibitors were also grown. All cell cultures were micro-injected with MBP-vp3 protein (produced and purified as described above) or MBP protein. In all cases when MBP-vp3 was micro-injected the majority of the Saos-2 cells underwent apoptosis 6 hours after micro-injection, whereas cells treated with one of the inhibitors did not undergo apoptosis, even at later time points. Independent of the presence of inhibitors, cells injected with MBP protein did not go into apoptosis.

These results indicate that apoptin-induced apoptosis is not inhibited by the transcription-inhibitors cycloheximide or actinomycin D and not by translation-inhibitors emetine or puromycin. Therefore, one can conclude that apoptin protein can induce apoptosis in human tumor cells without de novo synthesis of cellular proteins.

### 3.3 Apoptosis induction of the his-tagged NLS-apoptin fragment containing amino acids 1-69.

In the following experiments, we have examined whether a bacterially produced chimeric protein consisting of the N-terminal half of apoptin (amino acids 1-69) with at its N-terminus the nuclear localization signal (amino acids N-terminal-Proline-Proline-Lysine-Lysine-Lysine-Arginine-Lysine-Valine-C-terminal) of SV40 large T antigen and at its C-terminus a histidine tag, also reveals apoptotic activity in human tumor cells.

The used expression plasmid encoding the apoptin protein fragment NLS-vp3/1-69-H6 is shown in Figure 4b. The proteinaceous substance NLS-vp3/1-69-H6 was purified and produced as has been described for the above mentioned histidine-tagged apoptin (vp3) proteins.

Human Saos-2 cells were micro-injected with NLS-vp3/1-69-H6 protein as described for the above-mentioned micro-injected proteinaceous substances. As negative control, the human tumor cells were micro-injected with the non-apoptotic protein MBP and as positive control MBP-vp3 protein was micro-injected. Within 6 hours after micro-injection, the majority of the Saos-2 cells containing both MBP-vp3 and NLS-vp3/1-69-H6 protein became apoptotic, whereas the cells containing MBP protein did not.

Therefore, we conclude that proteinaceous substances containing the complete apoptin protein sequence or a specific apoptin protein fragment can induce apoptosis in human tumor cells.

### 4. TAT-apoptin

### 4.1 Transduction using denatured and refolded apoptin protein

### 4.1.1 Description of transduction-domain-apoptin construct

A DNA was constructed that encodes in frame for respectively a 6xHis-tag, a TAT-transduction domain, an HA-tag, followed by the coding sequence for apoptin and a stop codon. This construct was cloned in frame in a pet16b (Novagen) expression vector, which encodes for a 10x His-tag followed by the insert. The resulting construct is referred to as pETXNvp3, and the resulting protein will be referred to as XNvp3. See for the DNA sequence, figure 7 and for the protein sequence, figure 8.

### 4.1.2 Description of expression and purification

pETXNvp3 plasmid DNA was transformed into BL21(DE3)pLysS bacteria (Novagen) on LB plates containing the appropriate antibiotics, and an antibiotic resistant colony was grown to OD₆₀₀ of 0.6 in 1 liter LB plus antibiotics in a shaker flask. IPTG was added to 1 mM, and the cells were grown for an additional 3 hrs.

A bacterial pellet was obtained by centrifugation and sonicated on ice in 30 ml lysis buffer containing 150 mM NaCl and 0.5% Triton. After centrifugation the pellet was again sonicated as above, and inclusion bodies were centrifuged as a pellet. Expression of XNvp3 was confirmed by SDS-PAGE followed by Coomassie-blue staining and Westernblot analysis with 111.3, a vp3-protein specific monoclonal antibody.

The inclusion bodies were re-suspended in Nickel Loading Buffer (NLB;8M Urea, 1M NaCl, 50mM Phosphate buffer pH 7.5, 40mM imidazole) and loaded onto a 4 ml Ni-NTA column (Qiagen) equilibrated in the same buffer. The column was washed with 30 ml NLB, and washed with 10 ml MonoS Loading Buffer (MSLB;8M Urea, 250mM NaCl, 50mM phosphatebuffer pH 7.5) supplemented with 40mM imidazole. The bound proteins were eluted with MSLB supplemented with 250mM imidazole. The protein containing fractions were pooled and subsequently loaded on a 5 ml Mono-S chromatography column (Pharmacia) and washed with 2 column volumes MSLB. Refolding and elution was performed by washing the Mono-S column with 2M NaCl, 50mM phosphate buffer pH 7.5. The protein containing fractions were pooled and the buffer was exchanged on a PD10 column (Pharmacia) against DMEM or PBS. The protein was aliquoted and frozen at -80°C until further use.

### 4.1.3 Description of in-vitro transduction and specific killing of tumor cells

### Intracellular localization in tumor cells and normal cells.

To show that XNvp3 was able to penetrate into cells, and to assess its intracellular localization, the following experiment was performed. XNvp3 protein was added to cultured Saos-2 cells and VH10 cells at 50nM concentration in medium. After one hour the cells were fixed with 80% acetone, and the intracellular presence of XNvp3 protein was tested using antibodies 12CA5 (directed against the HA-tag) or 111.3 antibodies (directed against vp3 protein).

In both tumor and normal cells XNvp3 was located in the nucleus, as seen with confocal laser microscopy. This shows that XNvp3 can transduce into cells.

### Binding to Apoptin Associated Proteins in COS cells.

Next, we examined whether XNvp3 protein can bind to apoptin associated proteins as has been reported for apoptin protein (Noteborn and Danen-van Oorschot, 1999). To that end, the following experiment was performed. XNvp3 protein was incubated with COS cells that had been transfected 48 hours earlier with expression vectors encoding myc-tagged AAP1, which is one of the apoptin-associating proteins, or LacZ. Subsequently, the cells were lysed and an immuno-precipitation (IP) was performed with 12CA5. As a negative control, COS cells transfected with the same expression vectors but not incubated with XNvp3 were treated the same way. As a positive control, COS cells transfected with the same expression vectors together with an expression vector for HA-tagged vp3 were treated the same way. The IP's were analyzed for the presence of the myc-tagged proteins by SDS-PAGE and Western blotting with 9E10 (against the myc-tag).

The results show that XNvp3 can bind to an intracellular AAP in a similar fashion as apoptin (vp3) encoded by a transfected plasmid. Therefore, one can conclude that apoptin proteinaceous substances reveal essential biological activities of apoptin i.e. binding to its cellular counterparts.

### Induction of apoptosis in tumor cells versus normal cells

To show that XNvp3 can induce apoptosis in tumor cells, Saos-2 and VHSV-40 cells were cultured in the presence of 10 or 50nM XNvp3. To correct for possible degradation of XNvp3, the medium was replaced twice a day with fresh medium containing freshly thawed XNvp3. After four days the cells were fixed and the intracellular presence of XNvp3 and apoptosis were assessed with immuno-histochemistry as described before (Danen-Van Oorschot et al., 1997). The results show that XNvp3 can induce apoptosis in these cells at concentrations of around 10 to 50 nM.

A similar experiment was performed with non-transformed VH10 cells to establish the specificity of XNvp3. VH10 cells were cultured in medium containing 10 or 50 nM XNvp3, but also in a five time higher concentration at 250 nM. After four days, no significant apoptosis could be detected in the VH10 cells.

The same experiment was also performed with non-transformed keratinocytes and with primary mouse lymfocytes, and no apoptosis above background (medium alone) could be detected here either.

### Prevention of tumor cell growth in vitro

To extend the observation that XNvp3 can cause apoptosis specifically in tumor cells, Saos-2 cells, U2-OS cells, and VH10 cells were split 1:10 and cultured for two weeks in the presence of 50nM XNvp3 (replaced twice daily as described above) or in normal medium. The cells were then fixed with Methanol/acetic acid, and stained with Coomassie Blue. Although the cells in the control medium had all grown to confluency, the Saos-2 and U2OS cells treated with XNvp3 had all disappeared from the dish, indicating that they had undergone apoptosis. The VH10 cells treated with XNvp3 had grown to the same density as control treated VH10, showing that XNvp3 does not inhibit growth of non-transformed cells.

### 4.2 Transduction of non-denatured apoptin protein using cross-linked transduction domain peptides

### 4.2.1 Conjugation of TAT-peptide to MBP-vp3

MBPvp3 was purified as described for the above-mentioned micro-injection experiments. The protein was then chemically conjugated to synthetic TAT-peptide (aa 37-72, CFITKALGISYGRKKRRQRRPPQGSQTHQVSLSKQ) as described by Fawell et al. (1994). In short, the purified MBP-vp3 protein was activated with iodoacetamide, and desalted and concentrated. 4-(maleimidomethyl)-cyclohexanecarboxylic acid N-hydroxysuccinimide ester (SMCC) was added and after 30 minutes at room temperature the reaction was terminated by desalting on a G-25 column in 100 mM Na2HPO4 (pH 7.5). TAT peptide was added to the MBPbvp3-SMCC adduct and stored overnight at 4°C. The cross-linked conjugate was purified by size exclusion gel filtration and frozen at -80°C in small aliquots in the presence of 10% glycerol. A sample was analyzed by SDS PAGE using Coomassie-blue staining or Western blotting with antibodies raised against the TAT-peptide, or against vp3. The preparation was estimated to contain more than 50% conjugated MBP-vp3 protein, based on the relative intensity of the protein band with an apparent higher molecular weight on SDS-PAGE or the Western blot using anti-vp3 antibodies. The conjugated protein is referred to as MBP-vp3-TAT.

### 4.2.2. Description of in vitro transduction and specific killing of tumor cells

### Intracellular localization in tumor cells and normal cells.

To show that MBP-vp3-TAT was able to penetrate into cells, and to assess its intracellular localization, the following experiment was performed. MBP-vp3-TAT protein was added to cultured Saos-2 cells and VH10 cells at 5 microgram/ml concentration in medium. After 16 hours the cells were fixed by acetone fixation, and the intracellular presence of MBP-vp3-TAT was tested with antibodies directed against MBP (monoclonal mouse anti-MBP-clone R29; Zymed Laboratories, Inc. and polyclonal rabbit anti-MBP- clone C18/sc-808; Santa Cruz Biotechnology, Inc.) or 111.3 (reactive with vp3). In both tumor and normal cells MBP-vp3-TAT was located in the nucleus, as seen with confocal laser microscopy. This shows that MBP-vp3-TAT can transduce into cells.

### 4.2.3. Binding to Apoptin Associated Proteins in COS cells.

To show that MBP-vp3-TAT can bind to apoptin associated Proteins as well as transfected vp3 in cells, the following experiment was performed. MBP-vp3-TAT was incubated with COS cells that had been transfected 48 hours earlier with an expression vector encoding myc-tagged AAP-1 or LacZ. After 16 hours, the cells were lysed and anti MBP-vp3-TAT immunoprecipitation (IP) was performed with anti-MBP antibodies. As a negative control, COS cells transfected with the same expression vectors but not incubated with MBP-vp3-TAT were treated the same way. As a positive control, COS cells transfected with the same expression vectors together with an expression vector for HA-tagged vp3 were lysed and an IP was performed with anti-HA antibodies. The IP's were analyzed for the presence of the myc-tagged proteins by SDS-PAGE and Western blotting with 9E10 (against the myc-tag). The results show that MBP-vp3-TAT can bind to intracellular AAP's proving again that the apoptin proteinaceous substance contains the biological activity as seen for apoptin produced by transcription and translation of its DNA.

### 4.2.4. Induction of apoptosis in tumor cells versus normal cells

To show that MBP-vp3-TAT can induce apoptosis in tumor cells, Saos-2 and VHSV-40 cells were cultured in the presence of 1 or 5 microgram/ml MBP-vp3-TAT. To correct for possible degradation of MBP-vp3-TAT, the medium was replaced twice a day with fresh medium containing freshly thawed MBP-vp3-TAT. After four days the cells were fixed and the intracellular presence of MBP-vp3-TAT and apoptosis were assessed with immunohistochemistry as described before (Danen-Van Oorschot, 1997). The results show that MBP-vp3-TAT can induce apoptosis in these cells at concentrations of around 1 or 5 microgram/ml.

A similar experiment was performed with non-transformed VH10 cells to establish the specificity of MBP-vp3-TAT. VH10 cells were cultured in medium containing 1 or 5 microgram/ml MBP-vp3-TAT, but also in a five time higher concentration at 25 microgram/ml. After four days, no significant apoptosis could be detected in the VH10 cells.

The same experiment was also performed with non-transformed keratinocytes and with primary mouse lymphocytes, and no apoptosis above background (medium alone) could be detected here either.

### 4.2.5. Prevention of tumor cell growth in vitro

To extend the observation that MBP-vp3-TAT can cause apoptosis specifically in tumor cells, Saos-2 cells, U2-OS cells, and VH10 cells were split 1:10 and cultured for two weeks in the presence of 5 microgram/ml MBP-vp3-TAT (replaced twice daily as described above) or in normal medium. The cells were then fixed with Methanol/acetic acid, and stained with Coomassie Blue. Although the cells in the control medium had all grown to confluency, the Saos-2 and U2-OS cells treated with MBP-vp3-TAT had all disappeared from the dish, indicating that they had undergone apoptosis. The VH10 cells treated with MBP-vp3-TAT had grown to the same density as control treated VH10, showing that MBP-vp3-TAT does not inhibit growth of non-transformed cells.

### Description of the figures

Figure 1 shows the map of the MBP-vp3 fusion product inclusive the Asn-stretch and the thrombin cleavage site.

Figure 2 shows the partial DNA sequence of MBP-vp3. Underlined are the ATG-initiation codon of vp3 (apoptin) as well as the TAA-stop codon of vp3 (apoptin). The upstream sequences of the ATG-codon of vp3 are from the MBP construct showing that the apoptin sequence is in frame with the MBP sequence.

Figure 3 shows the protein sequence of MBP-vp3.

Figure 4a shows the map of vp3-H6, which is the complete apoptin (vp3) amino acid sequence containing 6 histidine residues at the C-terminal end.

Figure 4b shows the map of NLS-vp3/1-69-H6, which is made of the SV40 LT Nuclear Localization Signal (NLS), apoptin (vp3) amino-acid sequences 1-69 (1-69) and a C-terminal tag of 6 histidine residues.

Figure 5 shows the DNA sequence of plasmid encoding the vp3H6 construct. The NdeI and NotI sites are underlined in the shown DNA sequence. The vp3- and histidine-sequences are cloned in the NdeI and NotI sites of plasmid pET22b.

Figure 6 shows the protein sequence of the vp3H6 protein product.

Figure 7 shows the partial DNA sequence of pET-XNvp3 starting at the ATG of its NcoI site till the stop codon of apoptin (vp3).

Figure 8 shows the amino acid sequence of the XNvp3 protein product.

### REFERENCES

Bellamy, C.O.C., Malcomson, R.D.G., Harrison, D.J., and Wyllie, H. 1995. Cell death and disease: The biology and regulation of apoptosis. Seminars in Cancer Biology 6, 3-12.

Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.

Danen-Van Oorschot, A.A.A.M, Den Hollander, A., Takayama, S., Reed, J., Van der Eb, A.J. and Noteborn, M.H.M. (1997a). BAG-1 inhibits p53-induced but not apoptin-induced apoptosis. Apoptosis 2, 395-402.

Derossi, D., Chassaing, G., and Prochiantz, A. (1998). Trojan peptides: the penetratin system for intracellular delivery. Trends in Cell Biology 8, 84-87.

Duke, R.C., Ocjius, D.M., Young, J, D-E. (1996). Cell suicide in health and disease. Scientific American December 1996, 48-55.

Elliott, G. and O'Hare, P. (1997). Intercellular trafficking and protein delivery by a herpesvirus structural protein. Cell 88, 223-233.

Earnshaw, W.C., 1995. Nuclear changes in apoptosis. Current Opinion in Cell Biology 7, 337-343.

Fawell, S., Seery, J., Daikh, Y., Moore, C., Chen, L.,.L., Pepinsky, B., and Barsoum, J. Tat-mediated delivery of heterologous proteins into cells. Proceedings National Academic Sciences USA 91, 664-668.

Frankel, A.D., and Pabo, C.O. 1988. Cellular uptake of the Tat protein from human immunodeficiency virus. Cell 55, 1189-1193.

Green, M., and Loewenstein, P.M. 1988. Autonomous functional domains of chemically synthesized human immuno-deficiency virus Tat trans-activator protein. Cell 55, 1179-1188.

Hawiger, J. 1999. Non-invasive intracellular delivery of functional peptides and proteins. Current Opinion in Chemical Biology 3, 89-94.

Hockenberry, D.M. (1994). Bcl-2 in cancer, development and apoptosis. Journal of Cell Science, Supplement 18, 51-55.

Kerr, J.F.R., Winterford, C.M., and Harmon, B.V. (1994). Apoptosis: Its significance in cancer and cancer therapy. Cancer 73, 2013-2026.

Levine, A.J. (1997). p53, the cellular gatekeeper for growth and division. Cell 88, 323-331.

Lindgren,M. Haelbrink, M., Prochiantz, A., and Uelo Langel. Cell-penetrating peptides (2000). Trends in Pharmacological Sciences 21, 99-103.

McDonell T.J., Meyn, R.E., Robertson, L.E. (1995). Implications of apoptotic cell death regulation in cancer therapy. Seminars in Cancer Biology 6, 53-60.

Noteborn, M.H.M. (1996). PCT application WO 96/41191. Apoptin induces apoptosis in human transformed and malignant cells but not in normal cells as essential characteristic for the development of an anti-tumor therapy.

Noteborn, M.H.M., and De Boer, G.F. (1996). Patent USA/no. 030, 335.

Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.

Noteborn, M.H.M. and Danen-Van Oorschot. 1999. EP 99203465.2. AAP-1 in cytoplasma.

Noteborn, M.H.M., and Pietersen, A. (1998). A gene delivery vehicle expressing the apoptosis-inducing proteins VP2 and/or apoptin. PCT Application no. PCT/NL98/00213

Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.

Noteborn, M.H.M., and Zhang, Y. (1998). Methods and means for determining the transforming capability of agents, for determining the predisposition of cells to become transformed and prophylactic treatment of cancer using apoptin-like activity. PCT Application no. PCT/NL98/00457

Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.

Paulovich, A.G., Toczyski, D., Hartwell, H. (1997). When checkpoints fail. Cell 88, 315-321.

Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1999). Specific tumor-cell killing with adenovirus vectors containing the apoptin gene. Gene Therapy 6, 882-892.

Sachs, L. and Lotem, J. (1993). Control of programmed cell death in normal and leukemia cells: New implications for therapy. Blood 82, 15-21.

Sanger, F., Nicklen, S., and Coulsen, A.R. (1977). DNA sequencing with chain-terminating inhibitors. Proceedings National Academic Sciences USA 74, 5463-5467.

Steller, H. (1995). Mechanisms and genes of cellular suicide. Science 267, 1445-1449.

Schwarze, S.R. Ho, A. Vocero-Akbani, A., and Dowdy, S. (2000). In-vivo protein transduction: delivery of a biologically active protein into the mouse. Science 285, 1569-1572.

Telford, W.G., King, L.E., Fraker, P.J. (1992). Comparative evaluation of several DNA binding dyes in the detection of apoptosis-associated chromatin degradation by flow cytometry. Cytometry 13, 137-143.

Teodoro, J.G. and Branton, P.E. (1997). Regulation of apoptosis by viral gene products. Journal of Virology 71, 1739-1746.

Thompson, C.B. (1995). Apoptosis in the pathogenesis and treatment of disease. Science 267, 1456-1462.

Vives, E., Brodin, P., and Lebleu, B. (1997). A truncated HIV-1 Tat protein basic domain rapidly translocates through the plasma membrane and accumulates in the cell nucleus. Journal of Biological Chemistry 272, 16010-16017.

White, E. (1996). Life, death, and the pursuit of apoptosis. Genes and development 10, 1-15.

Wyllie, A.H. (1995). The genetic regulation of apoptosis. Current Opinion in Genetics and Development 5, 97-104.

Wyllie, A.H., Kerr, J.F.R., Currie, A.R. (1980). Cell death: The significance of apoptosis. International Review of Cytology 68, 251-306.

Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 S1, 118-120.

Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

## Claims

1. Apoptin or functional equivalent or functional fragment thereof provided with a means to deliver apoptin to a cell.

2. Apoptin according to claim 1 capable of providing tumor-specific apoptosis.

3. Apoptin according to claim 1 or 2 wherein said means comprises a protein transduction system.

4. Apoptin according to anyone of claims 1 to 3 wherein said means comprises the HIV TAT protein.

5. Apoptin according to anyone of claims 1 to 4 further provided with a fusion protein or tag.

6. Apoptin according to anyone of claims 1 to 5 which is non-denatured.

7. A nucleic acid encoding apoptin according to anyone of claims 1 to 6.

8. A vector comprising a nucleic acid according to claim 7.

9. A host cell comprising a nucleic acid according to claim 7 or a vector according to claim 8.

10. A host cell according to claim 9 which is a prokaryotic cell such as E.coli.

11. Use of a proteinaceous substance comprising apoptin or functional equivalent or functional fragment thereof for induction of tumor-specific apoptosis.

12. Use of a proteinaceous substance according to claim 11 wherein said substance further comprises a means to deliver said apoptin to a cell.

13. Use of a proteinaceous substance according to claim 12 wherein said means comprises a protein transduction system.

14. Use of a proteinaceous substance according to claim 12 or 13 wherein said means comprises the HIV TAT protein.

15. Use of a proteinaceous substance according to anyone of claims 11 to 14 further comprising a fusion protein or tag.

16. Use of a proteinaceous substance according to anyone of claims 11 to 15 wherein said proteinaceous substance is non-denatured.

17. Use according to anyone of claims 11 to 16 wherein said apoptosis is p53-independent.

18. A pharmaceutical composition comprising a proteinaceous substance comprising apoptin or functional equivalent or functional fragment thereof capable of providing tumor-specific apoptosis.

19. A pharmaceutical composition according to claim 18 wherein said proteinaceous substance further comprises a means to deliver said apoptin to a cell.

20. A pharmaceutical composition according to claim 19 wherein said means comprises a protein transduction system.

21. A pharmaceutical composition according to anyone of claims 19 or 20 wherein said means comprises the HIV TAT protein.

22. A pharmaceutical composition according to anyone of claims 18 to 21 wherein said proteinaceous substance further comprises a fusion protein or tag.

23. A pharmaceutical composition according to anyone of claims 18 to 22 wherein said proteinaceous substance is non-denatured.

24. A pharmaceutical composition according to anyone of claims 18 to 23 wherein said apoptosis is p53-independent.

25. Use of a proteinaceous substance comprising apoptin or functional equivalent or functional fragment thereof capable of providing apoptosis for the preparation of a pharmaceutical composition for the treatment of a disease where enhanced cell proliferation or decreased cell death is observed.

26. Use according to claim 25 wherein said proteinaceous substance further comprises a means to deliver said apoptin to a cell.

27. Use according to claim 26 wherein said means comprises a protein transduction system.

28. Use according to claim 26 or 27 wherein said means comprises the HIV TAT protein.

29. Use according to anyone of claims 25 to 28 wherein said proteinaceous substance further comprises a fusion protein or tag.

30. Use according to anyone of claims 25 to 29 wherein said proteinaceous substance is non-denatured.

31. Use according to anyone of claims 25 to 30 wherein said apoptosis is p53-independent.

32. Use according to anyone of claims 25 to 31 wherein said disease comprises cancer or auto-immune disease.

33. A method for detecting the presence of cancer cells or cells that are cancer prone in a sample of cells comprising providing cells in said sample with a proteinaceous substance comprising apoptin or functional equivalent or functional fragment thereof and determining the percentage of apoptosis of cells in said sample.

34. A method for detecting the presence of cancer cells or cells that are cancer prone in a sample of cells comprising providing cells in said sample with a proteinaceous substance comprising apoptin or functional equivalent or functional fragment thereof and determining the intracellular localization of the proteinaceous substance in cells in said sample.

35. A method according to claim 33 or 34 wherein said proteinaceous substance further comprises a means to deliver said apoptin to a cell.

36. A method according to claim 35 wherein said means comprises a protein transduction system.

37. A method according to claim 35 or 36 wherein said means comprises the HIV TAT protein.

38. A method according to anyone of claims 33 to 37wherein said proteinaceous substance further comprises a fusion protein or a tag.

39. A method according to anyone of claims 33 to 38 wherein said proteinaceous substance is non-denatured.
